# EUROPEAN PATENT SPECIFICATION

(11) **EP 1 511 516 B1**
(45) Date of publication and mention of the grant of the patent: **17.12.2008**
(21) Application number: 03755048.0
(22) Date of filing: 27.05.2003
(51) Int. Cl.: A61K 47/10, A61K 47/14, A61K 47/06, A61K 47/32, A61K 47/44, A61K 31/44

(54) **TOPICALLY APPLICABLE PHARMACEUTICAL PREPARATION**
TOPISCH ANWENDBARE PHARMAZEUTISCHE ZUBEREITUNG
PREPARATION PHARMACEUTIQUE TOPIQUE

(30) Priority: 28.05.2002 EP 02011830; 28.05.2002 DE 10223828; 14.03.2003 DE 10311613
(43) Date of publication of application: 09.03.2005
(60) Divisional application: 08166780.0
(73) Proprietor: Nycomed GmbH, 78467 Konstanz (DE)
(72) Inventor: BOLLE, Christina, 8280 Kreuzlingen (CH); LINDER, Rudolf, 78464 Konstanz (DE)
(74) Representative: Kratzer, Bernd
(86) International application number: PCT/EP2003/005524
(87) International publication number: WO 2003/099334

(56) References cited:
- WO-A-00/18388
- WO-A-01/32165
- WO-A-02/38155
- WO-A-95/01338
- US-A- 4 753 945
- US-A- 5 011 843
- US-B1- 6 174 878
- REID P: "ROFLUMILAST" CURRENT OPINION IN INVESTIGATIONAL DRUGS, CURRENT DRUGS, LONDON, GB, vol. 3, no. 8, August 2002 (2002-08), pages 1165-1170, XP001119630 ISSN: 0967-8298
- BRAUNWALD E, FAUCI AS, KASPER DL, HAUSER SL, LONGO DL, JAMESON JL: "Harrison's Principles of Internal Medicine (Chapter XIII: Endicronology and Metabolism) " 2001, MCGRAW-HILL, MEDICAL PUBLISHING DIVISION

## Description

### Technical field

The present invention relates to the field of pharmaceutical technology and describes a topically applicable pharmaceutical preparation comprising as active ingredient roflumilast. The invention additionally describes processes for producing the topically applicable pharmaceutical preparation. The invention particularly relates to the use for the treatment of dermatoses.

### Prior art

Cyclic nucleotide phosphodiesterase (PDE) inhibitors (specifically of type 4) are currently of special interest as a new generation of active ingredients for treating inflammatory disorders, especially disorders of the airways such as asthma or airway obstructions (such as, for example, COPD = chronic obstructive pulmonary disease). A number of PDE 4 inhibitors are currently undergoing advanced clinical testing, including a dosage form for oral administration comprising the active ingredient N-(3,5-dichloropyrid-4-yl)-3-cyclopropylmethoxy-4-difluoromethoxybenzamide (INN: roflumilast). This and other compounds with a benzamide structure and their use as cyclic nucleotide phosphodiesterase (PDE) inhibitors are described in WO 95/01338. These active ingredients are proposed in WO 95/01338 also for the treatment of certain disorders of the skin (such as, for example, dermatoses). WO 00/53182 proposes the use of roflumilast or its N-oxide for the treatment of multiple sclerosis.

For treating disorders of the skin it is desirable to provide the active pharmaceutical ingredient in a pharmaceutical preparation suitable for topical application. As the skilled person is aware, however, the provision of dosage forms for topical application may prove to be extremely difficult or is impossible if the intention is to administer an active ingredient which has a very low solubility. Thus, for example, the solubility in water found for the PDE 4 Inhibitor N-(3,5-dichloropyrid-4-yl)-3-cyclopropylmethoxy-4-difluoromethoxybenzamide (INN: roflumilast), which is described in WO 95/01338, is only 0.53 mg/l at 21°C.

### Description of the invention

It has now been found, surprisingly, that topically applicable pharmaceutical preparations comprising the slightly soluble PDE 4 inhibitor roflumilast show a very good effect in the treatment of dermatoses on local, dermal application. Also found, entirely surprisingly, besides the local effect, is an excellent systemic effect which is comparable with that of an oral dosage form.

The invention discloses in a first aspect a pharmaceutical preparation which can be administered topically and comprises an active pharmaceutical ingredient together with one or more pharmaceutical carriers and/or excipients suitable for topical administration, the active pharmaceutical ingredient being a compound selected from the group consisting of roflumilast, salts of roflumilast, the N-oxide of roflumilast and salts thereof.

Roflumilast is the INN for a compound of the formula I 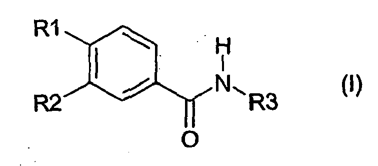 in which
- R1: is difluoromethoxy,
- R2: is cyclopropylmethoxy and
- R3: is 3,5-dichloropyrid-4-yl.

This compound has the chemical name N-(3,5-dichloropyrld-4-yl)-3-cyclopropylmethoxy-4-difluoromethoxybenzamide (INN: roflumilast). The N-oxide of roflumilast has the chemical name 3-cyclopropylmethoxy-4-difluoromethoxy-N-(3,5-dichloropyrid-4-yl 1-oxide)benzamide.

This compound of the formula I, its salts, the N-oxide, its salts and the use of these compounds as phosphodiesterase (PDE) 4 inhibitors are described in the international patent application WO 95/01338.

Salts suitable for compounds of the formula I - depending on the substitution - are all acid addition salts but, in particular, all salts with bases. Particular mention may be made of the pharmacologically acceptable salts of the inorganic and organic acids and bases normality used in pharmaceutical technology. Pharmacologically unacceptable salts which, for example, may be the initial products of the process for preparing the compounds of the invention on the industrial scale are converted into pharmacologically acceptable salts by processes known to the skilled worker. Those suitable on the one hand are water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid, or 3-hydroxy-2-naphthoic acid, the acids being employed to prepare the salts in the equimolar ratio of amounts, or one differing therefrom - depending on whether the acid is monobasic or polybasic and depending on which salt is desired.

On the other hand, salts with bases are also particularly suitable. Examples of basic salts which may be mentioned are lithium, sodium, potassium, calcium, aluminium, magnesium, titanium, ammonium, meglumine or guanidinium salts, once again the bases being employed to prepare the salts in the equimolar ratio of amounts or one differing therefrom.

The proportion (in per cent by weight based on the weight of the finished pharmaceutical preparation; w/w) of active pharmaceutical ingredient in the pharmaceutical preparation of the invention is usually from 0.001 to 50% by weight. The proportion of active pharmaceutical ingredient is preferably up to 1% by weight.

The pharmaceutical carriers and/or excipients suitable for topical administration are preferably according to the disclosure of the invention conventional carriers and/or excipients known to the skilled person in connection with pharmaceutical preparations for dermal administration (= dermatologicals). Examples which may be mentioned are carriers and/or excipients which are suitable for producing dusting powders, emulsions, suspensions, sprays, oils, ointments, greasy ointments, creams, pastes, gels, foams or solutions, and transdermal therapeutic systems.

The topical pharmaceutical preparation disclosed in the present invention can be produced by processes familiar to the skilled person.

Conventional dermatologicals and their production, and preferred carriers and/or excipients for the individual pharmaceutical preparations are described, for example, in the textbook "Pharmazeutische Technologie" (Sucker, Fuchs, Speiser, Georg Thieme Verlag,1978 from page 629).

The invention discloses a first embodiment, wherein the topical pharmaceutical preparation is a semisolid dosage form. Examples which may be mentioned are, in particular, ointments (e.g. solution ointment, suspension ointment), creams, gels or pastes.

Oil-in-water or water-in-oil emulsions are normally referred to as creams. Chiefly used for the oily phase are fatty alcohols, e.g. lauryl, cetyl or stearyl alcohol, fatty acids, e.g. palmitic or stearic acid, liquid or solid paraffins or ozokerite, liquid to solid waxes, e.g. isopropyl myristate, natural or partially synthetic fat, e.g. coconut fatty acid triglyceride, hardened oils, e.g. hydrogenated peanut or castor oil, or fatty acid partial esters of glycerol, e.g. glycerol monostearate or glycerol distearate. Suitable emulsifiers are surface-active substances, e.g. nonionic surfactants, e.g. fatty acid esters of polyalcohols or ethylene oxide adducts thereof, such as polyglycerol fatty acid esters or polyoxyethylene sorbitan fatty acid esters (Tween®: ICI,) sorbitan fatty acid esters (Span®: ICI), such as, for example, sorbitan oleate and/or sorbitan isostearate, sterols, also polyoxyethylene fatty alcohol ethers or fatty acid esters, or anionic surfactants such as alkali metal salts of fatty alcohol sulphates, e.g. sodium lauryl sulphate, sodium cetyl sulphate or sodium stearyl sulphate, which are normally used in the presence of said fatty alcohols, e.g. cetyl alcohol or stearyl alcohol. It is possible to add to the aqueous phase inter alia agents which prevent the cream drying out, e.g. polyalcohols such as glycerol, sorbitol, propylene glycol and/or polyethylene glycols, also preservatives, fragrances etc.

Ointments may be anhydrous and contain as base the paraffins which are suitable for topical use and are liquid at body temperature, especially low-viscosity paraffin, also the said natural or partially synthetic fats, e.g. coconut fatty acid triglyceride, hardened oils, e.g. hydrogenated peanut or castor oil, fatty acid partial esters of glycerol, e.g. glycerol monostearate and distearate, silicones, e.g. polydimethylsiloxanes, e.g. hexamethyldisiloxane or octamethyltrisiloxane, and, for example, the fatty alcohols mentioned in connection with the hydrous creams and increasing the water uptake capacity, and sterols, wool waxes, other emulsifiers and/or other additives.

In the case of gels, a distinction is made between hydrous, anhydrous and low water-content gels, which consist of swellable, get-forming material. Chiefly suitable are transparent hydrogels based on inorganic or organic macromolecules. Macromolecular inorganic components with gel-forming properties are predominantly hydrous or water-absorbing silicates such as aluminium silicates, e.g. bentonite, magnesium-aluminium silicates, e.g. Veegum® - Vanderbilt Exp. Corp., or colloidal silica, e.g. Aerosil® - Degussa. Examples of macromolecular organic substances used are natural, semisynthetic or synthetic polymers. Natural and semisynthetic polymers are derived, for example, from polysaccharides with different carbohydrate units, such as cellulose, starch, tragacanth, gum arabic, agar-agar, gelatin, alginic acid and salts thereof, e.g. sodium alginate and derivatives thereof, lower alkylcellulose, for example methyl- or ethylcellulose, carboxy- or hydroxy-lower-alkylcellulose, e.g. carboxymethyl- or hydroxypropylcellulose. The units of synthetic, get-forming polymers are, for example, unsaturated, substituted aliphatic compounds such as vinyl alcohol, vinylpyrrolidone, acrylic or methacrylic acid. Examples to be mentioned of such polymers are polyvinyl alcohol derivatives such as Polyviol®- Wacker, polyvinylpyrrolidones, such as Kollidon® - BASF or Polyplasdon® - General Aniline, polyacrylates and polymethacrylates, such as Rohagit S® - Rohm und Haas. It is possible to add conventional additives such as preservatives or fragrances to the gels.

Pastes are creams or ointments with the constituents mentioned hereinbefore and secretion-absorbing dusting powder constituents such as metal oxides, e.g. titanium oxide or zinc oxide, also talc and/or aluminium silicates, which have the task of binding moisture or secretions.

The invention discloses a preferred embodiment, wherein the topical pharmaceutical preparation is a semisolid pharmaceutical preparation, with one of the excipients being polyethylene glycol, in particular polyethylene glycol 400.

The invention discloses in further embodiment, wherein the topical pharmaceutical preparation is a transdermal therapeutic system (TTSs), for example a system as described in Pharmazeutische Technologie: Moderne Arzneiformen, Wissenschaftliche Verlagsgesellschaft mbH Stuttgart 1997, pages 81 et sec. TTSs are characterized in principle by a defined supply of medicinal substance to the skin, a total dose of the medicinal substance in the TTS, a total area and an area which is possibly different therefrom for release of the medicinal substance, a covering sheet (backing layer) which is impermeable to the medicinal substance, a medicinal substance reservoir, a control element which controls the supply of medicinal substance to the skin, a (pressure-sensitive) adhesive layer and a detachable protective layer. It is possible on occasions for more than one function to be fulfilled by one and the same element, e.g. reservoir, control and adhesive functions by a suitable adhesive matrix. From the viewpoint of pharmaceutical technology, TTSs are categorized according to the way the control function is achieved, that is to say how it controls the supply of medicinal substance to the skin. Examples which are mentioned here are TTSs with membrane permeation-controlled release (membrane moderated drug delivery), TTSs with matrix diffusion-controlled release and TTSs with microreservoir solution-controlled release.

TTSs with membrane permeation-controlled release are characterized by a polymer membrane composed of a PVA-VA copolymer (Chronomer®) which controls the permeation of the medicinal substance from the reservoir into the skin. The medicinal substance is initially in the form of solid particles or as a dispersion or solution in the reservoir. The polymer membrane can be attached to the reservoir in various ways (extrusion, encapsulation, microencapsulation). TTSs with matrix diffusion-controlled release have a comparatively simpler structure. They contain no separate control element. The release of medicinal substance is controlled by a lipophilic or hydrophilic polymer matrix and/or the adhesive layer. It is possible to distinguish, according to the characteristics of the matrix, between TTSs with a matrix in gel form and TTSs which represent solid polymer laminates. The medicinal substance reservoir is formed by the medicinal substance dissolved in the matrix (monolithic system) or a homogeneous dispersion of solid medicinal substance particles. A matrix TTS can be produced by mixing the medicinal substance particles with a viscous liquid or semisolid polymer at room temperature, followed by crosslinking the polymer chains. A further possibility is also to mix the medicinal substance at elevated temperature with softened polymer (hot melt technique), or the two components (dissolved in an organic solvent) are mixed together and the solvent is then removed in vacuo (solvent evaporation). Shaping is possible by pouring into suitable moulds, spreading with special devices (knives) or by extrusion. In the case of TTSs with microreservoir solution-controlled release (microsealed drug delivery, MDD principle), numerous microcompartments containing the active ingredient and 10-200 µm in size are embedded in a matrix which represents both reservoir and delivery-control element. Because of the matrix, these TTSs are actually assigned to the matrix systems. For production, the medicinal substance is initially dispersed together with water and 40% polyethylene glycol 400 in isopropyl palmitate, which acts as permeation promoter. The resulting dispersion is incorporated by using a special high-energy dispersion technique into a viscous silicone elastomer which simultaneously undergoes catalytic polymerization. The medicinal substance-containing matrix can be shaped specifically by melt or extrusion techniques before it is combined with the carrier in the manner already described. Depending on the physicochemical properties of the medicinal substances and the intended liberation, it is possible to cover the matrix with a layer of a biocompatible polymer in order thus to modify the mechanism and the rate of liberation.

The invention discloses another embodiment, wherein the topical pharmaceutical preparation is a dosage form for use on the eye (ophthalmologicals). Examples which may be mentioned in this connection are eyebaths or eye lotions, eye inserts, eye ointments, eye sprays, eye drops, preparations for intraocular injection and eyelid ointments. The invention discloses in a preferred embodiment, the dosage form which is an eye ointment or eye drops. Eye drops preferably comprise according to the invention aqueous or oily suspensions of the active ingredient. It is preferred in this connection for the particle size of the active ingredient employed to be 90% less than 10 µm.

Preferably used in the case of aqueous suspensions are suspension stabilizers such as, for example, substituted celluloses (e.g. methylcellulose, hydroxypropylmethylcellulose), polyvinyl alcohol, polyvinylpyrrolidone, In addition to preservatives (e.g. chlorocresol, phenylmercury compounds, phenylethanol, benzalkonium chloride or mixtures of individual components) and, where appropriate, sodium chloride to adjust to isotonicity. Preferably employed in the case of oily eye drops are castor oil, peanut oil or medium chain length triglycerides. It is possible in the case of eye ointments to use according to the invention ointment bases which have the following properties: sterility or extremely low microbe content, non-irritating, good activity, good distribution of the active ingredient or its solution in the ointment, suppleness, rapid dispersion as fine film over the eyeball, good adhesion to the eye, good stability and low impairment of vision. Hydrocarbon- or cholesterol-containing bases will therefore preferably be employed for eye ointments. In the case of petrolatum, liquid paraffin is preferably added for consistency reasons. To achieve good spreading, it is preferred to provide compositions of limited viscosity. The viscosity at 32°C is preferably below 1 000 mPa.s, and the yield point is preferably below 300 mPa. In the case of suspension ointments It is preferred according to the invention for 90% of the active ingredient particles to be below 10 µm, and no particles above 90 µm should occur. In the case of water/oil emulsion ointments, it is preferred according to the invention to add preservatives such as benzalkonium chloride, thiomersal or phenylethyl alcohol.

### Examples

### Production of the dosage forms

### Example 1

| 550 grams contain | |
|---|---|
| | |
| Polyethylene glycol 400 | 440.00 g |
| Carbopol 934® | 8.25 g |
| Roflumilast | 1.375 g |
| Sodium hydroxide solution q.s. | |
| Purified water | to 550.00 g |

Production takes place by dissolving the active ingredient in the stated amount of polyethylene glycol at about 60-70°C. About 90 grams of purified water are added and mixed homogeneously, and the Carbopol 934 is homogeneously dispersed therein with a high-speed stirrer. While stirring slowly, sodium hydroxide solution is added until a pH of 6.5-7.5 is reached. The remaining water is added up to the final weight and homogeneously mixed.

### Example 2

| 550 grams contain | |
|---|---|
| | |
| Roflumilast | 1.65 g |
| Polyethylene glycol 400 | 440.00 g |
| Polyethylene glycol 4000 | to 550.0 g |

Production takes place by treating the two polyethylene glycols to 70°C to give a clear melt. The active ingredient is added likewise to give a clear solution. The preparation is cooled to room temperature while stirring slowly.

### Example 3

| 550 grams contain | |
|---|---|
| | |
| Roflumilast | 1.10 g |
| Tego Care 150® | 27.50 g |
| (Th. Goldschmidt) | |
| Neutral oil (Miglyol 812®) | 137.50 g |
| Polyethylene glycol 400 | 275.00 g |
| Cetostearyl alcohol | 11.00 g |
| Purified water | to 550 g |

Production takes place by making a clear solution of the neutral oil, the cetostearyl alcohol and Tego Care 150 at about 70°C. The polyethylene glycol, In which the roflumilast has been dissolved, is likewise stirred in using a high-speed stirrer. The water heated to 70°C is added to the lipid phase. A Turrax is used for homogenization. The preparation is then stirred until cold (room temperature).

### Example 4

| 100 grams contain | |
|---|---|
| | |
| Roflumilast | 0.25 g |
| Neutral oil (Miglyol 812®) | 16.00 g |
| Glycerol monostearate | 8.00 g |
| Cremophor A6® (BASF) | 4.00 g |
| Polyethylene glycol 400 | 62.50 g |
| Purified water | to 100.00 g |

Production takes place by heating all the components (apart from water) together to about 70-80°C to give a clear solution. The water is then added while stirring, and the preparation produced in this way is cooled to room temperature while stirring.

### Example 5

| 100 grams contain | |
|---|---|
| | |
| Roflumilast | 0.25 g |
| Liquid paraffin | 15.00 g |
| Wool wax | 5.00 g |
| White petrolatum | to 100 g |

Production takes place by making a clear melt of the liquid paraffin, the wool wax and the white petrolatum at about 80°C. The micronized active ingredient is added, and the preparation is stirred until it has cooled to room temperature.

### Example 6

| | |
|---|---|
| Roflumilast | 0.10 g |
| Liquid paraffin | 10.00 g |
| Wool wax | 5.00 g |
| White petrolatum | to 100 g |

Production takes place in analogy to Example 5.

### Example 7

| | |
|---|---|
| Roflumilast | 0.10 g |
| Neutral oil (Miglyol 812®) | 16.00 g |
| Glycerol monostearate | 8.00 g |
| Cremophor A6® (BASF) | 2.00 g |
| Polyethylene glycol 400 | 62.50 g |
| Purified water | to 100.00 g |

Production takes place in analogy to Example 4.

### Example 8

| | |
|---|---|
| Roflumilast | 0.10 g |
| Neutral oil (Miglyol 812®) | 16.00 g |
| Glycerol monostearate | 8.00 g |
| Cremophor A6® (BASF) | 4.00 g |
| Polyethylene glycol 400 | 62.50 g |
| Purified water | to 100.00 g |

Production takes place in analogy to Example 4.

### Example 9

**Composition of an eye ointment (quantity for 1 000 grams)**

| | |
|---|---|
| Roflumilast | 1 g |
| Cetyl alcohol | 4 g |
| High-viscosity paraffin | 200 g |
| White petrolatum | 795 g |

Production: A clear melt of the cetyl alcohol, the high-viscosity paraffin and the white petrolatum is prepared at about 70°C. The micronized roflumilast (90% of the particles below 10 µm) is stirred in, and a homogeneous dispersion is prepared using an Ultra-Turrax. The suspension is cooled to room temperature while stirring and used to fill suitable tubes.

### Example 10

**Composition of a drop solution in the form of an emulsion (quantity for 1 000 millilitres)**

| | |
|---|---|
| Roflumilast | 1.5 g |
| Medium chain length triglycerides | 100.0 g |
| Lecithin | 12.0 g |
| Glycerol | 25.0 g |
| Thiomersal | 0.1 g |
| Purified water | to 1 000 ml |

Production: First the roflumilast and then the lecithin are dissolved in the medium chain length triglycerides and the glycerol at 30°C-40°C. While stirring vigorously, the purified water is added and then homogenized until the droplet size of the disperse phase is below 500 nm. The thiomersal is dissolved by stirring. The emulsion is filtered through a 0.45 µm filter and dispensed into suitable containers.

### Example 11

**Composition of a nose ointment (quantity for 1 000 grams)**

| | |
|---|---|
| Roflumilast | 1 g |
| Cetyl alcohol | 4 g |
| Wool wax | 50 g |
| High-viscosity paraffin | 200 g |
| White petrolatum | 745 g |

Production: A clear melt of the cetyl alcohol, the high-viscosity paraffin, the wool wax and the white petrolatum is prepared at about 70°C. The micronized roflumilast (90% of the particles below 10 µm) is stirred in, and a homogeneous dispersion is prepared using an Ultra-Turrax. The suspension is cooled to room temperature while stirring and used to fill suitable tubes.

### Investigations of the pharmacokinetics of the topical pharmaceutical preparations

### Comparison of pharmacokinetics parameters of topical pharmaceutical preparations disclosed in the present invention with oral form

### Example A

A preparation corresponding to Example 7 and a preparation corresponding to Example 8 containing [¹⁴C]roflumilast were applied to shaven areas of rat skin (5 male Wistar rats) 4 cm² in size. The radioactivity concentrations were measured in the plasma after 1 h, 4 h, 8 h, 24 h and in the urine (0-24 h) (n = 5). The dose was 1.7 mg/kg.

### Results:

Preparation of Example 7: Cmax: 0.214 mg equiv./l, AUC(0-24 h): 4.13 (mg equiv./l × h)
Preparation of Example 8: Cmax: 0.214 mg equiv./l, AUC(0-24 h): 3.99 (mg equiv./l × h)

The results standardized to 1 mg/kg are
Preparation of Example 7: Cmax: 0.126, AUC: 2.43
Preparation of Example 8: Cmax: 0.126, AUC: 2.35

Comparison with kinetic parameters after oral administration of 1 mg/kg:
Cmax: 0.225 mg equiv./l, AUC(0-24 h): 3.10 (mg equiv./l × h)

The ratio of the AUC (preparation of Example 7) to the AUC (oral) is 78% and that of the AUC (preparation of Example 8) to the AUC (oral) is 76%.

Results of comparison of the excretions with the urine:
Preparation of Example 7: 19.4% of the dose
Preparation of Example 8: 18.0% of the dose
Oral administration: 18.4% of the dose

### Conclusion:

After percutaneous administration of 1.7 mg/kg [¹⁴C]roflumilast to rats, the total radioactivity is transported well through the skin and reaches a maximum plasma level of 0.214 mg equiv./l after 4 h, irrespective of the preparation employed. Based on the total radioactivity, the AUCs and the excretions with the urine after percutaneous administration are negligibly different from those after oral administration.

### Example B

A preparation corresponding to Example 5 containing [¹⁴C]roflumilast was applied to a shaven area of rat skin (male Wistar rat) 4 cm² in size. The radioactivity concentrations were measured in the plasma after 1 h, 4 h, 8 h, 24 h and In the urine (0-24 h) (n = 5). The dose was 1.77 mg/kg.

### Preparation of Example 5: Cmax: 0.331 mg equiv./l, AUC(0-24 h): 4.99 (mg equiv./l × h)

The results standardized to 1 mg/kg are
Preparation of Example 5: Cmax: 0.187, AUC: 2.82

Comparison with kinetic parameters after oral administration of 1 mg/kg:
Cmax: 0.225 mg equiv./l. AUC(0-24 h): 3.10 (mg equiv./l × h)

Results of comparison of the excretions with the urine:
Preparation of Example 5: 22.0% of the dose
Oral administration: 18.4% of the dose

### Conclusion:

These data show that roflumilast is absorbed from the preparation of Example 5 even somewhat better than from the preparations corresponding to Example 7 or 8. The excretion with the urine in the 24 h after administration is 22%, which is also in the region of the excretion with the urine after dermal administration of the preparations corresponding to Example 7 or 8. Comparison with oral administration shows that, irrespective of the composition of the topical preparation, similar Cmax and AUCs and similar excretions with the urine are achieved.

### Industrial applicability

The pharmaceutical preparations disclosed in the present invention are particularly suitable for the treatment of dermatoses (especially of a proliferative, inflammatory and allergic nature) selected from the group consisting of psoriasis (vutgaris), toxic and allergic contact eczema, atopic eczema, seborrhoeic eczema, lichen simplex, sunburn, pruritus in the genitoanal region, alopecia areata, hypertrophic scars, discoid lupus erythematosus, follicular and extensive pyodermas, endogenous and exogenous acne, acne rosacea and other proliferative, inflammatory and allergic skin disorders. Mention may preferably be made of the use of the pharmaceutical preparations disclosed in the present invention in the treatment of psoriasis and atopic eczema.

The invention therefore relates to the use of roflumilast, salts of roflumilast, the N-oxide of the pyridine residue of roflumilast or salts thereof for producing a topical pharmaceutical preparation for dermal administration for the treatment of a dermatose selected from the group consisting of psoriasis (vulgaris), toxic and allergic contact eczema, atopic eczema, seborrhoeic eczema, lichen simplex, sunburn, pruritus in the genitoanal region, alopecia areata, hypertrophic scars, discoid lupus erythematosus, follicular and extensive pyodermas, endogenous and exogenous acne, acne rosacea and other proliferative, inflammatory and allergic skin disorders.

The invention further discloses a method for the treatment of mammals, including humans, suffering from one of the abovementioned diseases. The method is characterized in that a therapeutically effective and pharmacologically suitable amount of an active pharmaceutical ingredient selected from the group of compounds roflumilast, salts of roflumilast, the N-oxide of roflumilast and salts thereof is administered to the mammal with the disease, with the active pharmaceutical ingredient being administered in a topical pharmaceutical preparation disclosed in the invention. The disease is a dermatose selected from the group consisting of psoriasis (vulgaris), toxic and allergic contact eczema, atopic eczema, seborrhoeic eczema, lichen simplex, sunburn, pruritus in the genitoanal region, alopecia areata, hypertrophic scars, discoid lupus erythematosus, follicular and extensive pyodermas, endogenous and exogenous acne, acne rosacea and other proliferative, inflammatory and allergic skin disorders. The method is characterized in that the administration takes place by dermal administration, i.e. through application of the topical pharmaceutical preparations disclosed in the invention to the skin or mucous membranes.

The dosage forms disclosed in the present invention comprise the active pharmaceutical ingredient in the dose customary for the treatment of the particular disease. The dosage of the active ingredient is of the order of magnitude customary for PDE inhibitors, it being possible to administer the daily dose in one or more dosage units. Customary dosages are disclosed for example in WO 95/01338. The normal dose on systemic therapy (oral) is between 0.001 and 3 mg per kilogram and day. Dosage forms preferred according to the disclosure of the invention for topical administration contain from 0.005 mg to 5 mg of roflumilast, preferably from 0.01 mg to 2.5 mg, particularly preferably 0.1 mg to 0.5 mg of roflumilast per dosage unit. Examples of pharmaceutical preparations disclosed in the present invention contain 0.01 mg, 0.1 mg, 0.125 mg, 0.25 mg and 0.5 mg of roflumilast per dosage unit.

## Claims

1. Use of a compound selected from the group consisting of raflumilast, salts of roflumilast, the N-oxide of the pyridine residue of roflumilast and salts thereof in the manufacture of a topically applicable pharmaceutical preparation for dermal administration for the treatment of a dermatosis selected from the group of psoriasis (vulgaris), toxic and allergic contact eczema, atopic eczema, seborrhoeic eczema, lichen simplex, sunburn, pruritus in the genitoanal region, alopecia areata, hypertrophic scars, discoid lupus erythematosus, follicular and extensive pyodermas, endogenous and exogenous acne, acne rosacea or other proliferative, inflammatory and allergic skin disorders.

2. Use according to Claim 1, where roflumilast is a compound of the formula I 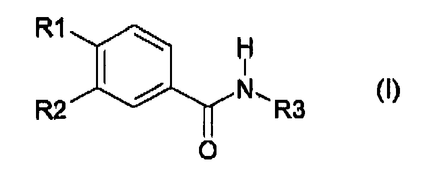 in which
R1 is difluoromethoxy,
R2 is cyclopropylmethoxy and
R3 is 3,5-dichloropyrid-4-yl.

3. Use according to Claim 1, wherein the topically applicable pharmaceutical preparation is a semi-solid dosage form selected from the group of ointments (e.g. solution ointment, suspension ointment), creams, gels or pastes.

4. Use according to Claim 1, wherein the topically applicable pharmaceutical preparation is a transdermal therapeutic system (TTS).

5. Use according to Claim 1 wherein the topically applicable pharmaceutical preparation is a semisolid pharmaceutical preparation, with one of the excipients being polyethylene glycol.

6. Use according to Claim 5, wherein the polyethylene glycol is polyethylene glycol 400.

7. Use according to Claim 1. wherein the topically applicable pharmaceutical preparation is an ointment and which contains a paraffin which Is suitable for topical use and is liquid at body temperature.

## Patentansprüche

1. Verwendung einer Verbindung, ausgewählt aus der Gruppe, bestehend aus Roflumilast, Salzen von Roflumilast, dem N-Oxid des Pyridinrestes von Roflumilast und Salzen davon, zur Herstellung einer topisch applizierbaren Arzneimittelzubereitung für die dermale Applikation zur Behandlung einer Dermatose, bei der es sich um Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea oder andere proliferative, entzündliche und allergische Hautkrankheiten handelt.

2. Verwendung nach Anspruch 1, bei der es sich bei Roflumilast um eine Verbindung der Formel I handelt, 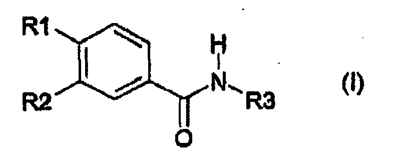 worin
R1 Difluormethoxy,
R2 Cyclopropylmethoxy und
R3 3,5-Dichlorpyrid-4-yl bedeutet.

3. Verwendung nach Anspruch 1, bei der es sich bei der topisch applizierbaren Arzneimittelzubereitung um eine halbfeste Darreichungsform handelt, ausgewählt aus der Gruppe Salben (z.B. Lösungssalbe, Suspensionssalbe), Cremes, Gele oder Pasten.

4. Verwendung nach Anspruch 1, bei der es sich bei der topisch applizierbaren Arzneimittelzubereitung um ein transdermales therapeutisches System (TTS) handelt.

5. Verwendung nach Anspruch 1, bei der es sich bei der topisch applizierbaren Arzneimittelzubereitung um eine halbfeste Arzneimittelzubereitung handelt, bei der es sich bei einem der Hilfsstoffe um Polyethylenglykol handelt.

6. Verwendung nach Anspruch 5, bei der es sich bei dem Polyethylenglykol um Polyethylenglykol 400 handelt.

7. Verwendung nach Anspruch 1, bei der es sich bei der topisch applizierbaren Arzneimittelzubereitung um eine Salbe handelt, die ein für die topische Anwendung geeignetes, bei Körpertemperatur flüssiges Paraffin enthält.

## Revendications

1. Utilisation d'un composé choisi dans le groupe constitué du roflumilast, de sels de roflumilast, du N-oxyde du résidu pyridine du roflumilast et des sels de celui-ci, pour la fabrication d'une préparation pharmaceutique applicable par voie topique destinée à une administration dermique pour le traitement d'une dermatose choisie dans le groupe constitué du psoriasis (vulgaire), de l'eczéma de contact toxique et allergique, de l'eczéma atopique, de l'eczéma séborrhéique, du lichen simplex, d'un érythème solaire, d'un prurit dans la région génito-anale, de la pelade, de cicatrices hypertrophiques, d'un lupus érythémateux discoïde, de pyodermites folliculaires et étendues, de l'acné endogène et exogène, de l'acné rosacée ou d'autres troubles cutanés prolifératifs, inflammatoires et allergiques.

2. Utilisation selon la revendication 1, selon laquelle le roflumilast est un composé de formule I 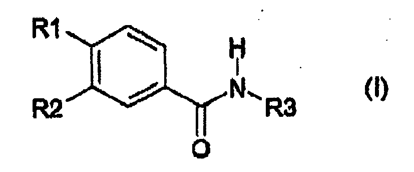 dans laquelle
R1 est difluorométhoxy,
R2 est cyclopropylméthoxy et
R3 est 3,5-dichloropyrid-4-yle.

3. Utilisation selon la revendication 1, selon laquelle la préparation pharmaceutique applicable par voie topique est une forme galénique semi-solide choisie dans le groupe des pommades (par exemple une pommade-solution, une pommade-suspension), des crèmes, des gels ou des pâtes.

4. Utilisation selon la revendication 1, selon laquelle la préparation pharmaceutique applicable par voie topique est un système thérapeutique transdermique (TTS).

5. Utilisation selon la revendication 1, selon laquelle la préparation pharmaceutique applicable par voie topique est une préparation pharmaceutique semi-solide, l'un des excipients étant le polyéthylène glycol.

6. Utilisation selon la revendication 5, selon laquelle le polyéthylène glycol est le polyéthylène glycol 400.

7. Utilisation selon la revendication 1, selon laquelle la préparation pharmaceutique applicable par voie topique est une pommade qui contient une paraffine qui est appropriée pour une utilisation topique et qui est liquide à la température du corps.
